# EUROPEAN PATENT APPLICATION

(11) **EP 4 417 962 A1**
(43) Date of publication of application: **21.08.2024**
(21) Application number: 23743254.7
(22) Date of filing: 17.01.2023
(51) Int. Cl.: G01N 15/02, G01N 33/22

(54) **PARTICLE SIZE ESTIMATION METHOD AND PARTICLE SIZE ESTIMATION DEVICE**

(30) Priority: 20.01.2022 JP 2022007397
(71) Applicant: JFE Steel Corporation, Tokyo 100-0011 (JP)
(72) Inventor: MIZUNO Takuyo, Tokyo 100-0011 (JP); YAMAHIRA Naoshi, Tokyo 100-0011 (JP)
(74) Representative: Hoffmann Eitle
(86) International application number: PCT/JP2023/001226
(87) International publication number: WO 2023/140260

(57) **Abstract**

A particle size estimation device 10 includes an acoustic sensor 14 and an information processing unit 15. The acoustic sensor 14 is located in the vicinity of predetermined equipment 11. The predetermined equipment 11 handles a particle to be measured. The acoustic sensor 14 collects sound of collision when the particle collides with the predetermined equipment 11, wherein the particle includes a plurality of particles. The information processing unit 15 makes an estimation regarding the particle size of the particles, based on the sound collected by the acoustic sensor 14.

## Description

### CROSS-REFERENCE TO RELATED APPLICATION

This application claims priority to Japanese Patent Application No. 2022-007397, filed on January 10, 2022, the entire contents of which are incorporated herein by reference.

### TECHNICAL FIELD

The present disclosure relates to a particle size estimation method and a particle size estimation device.

### BACKGROUND

In steelmaking processes, coke in blast furnaces is required to have high strength and uniformity to ensure gas permeability in the furnaces. To produce uniform and high-strength coke, the particle size of coal is adjusted to an ideal particle size ratio so as to increase the bulk density of the coal charged into coke ovens so that firm contact occurs between particles when the coal is heated and dry-distilled in the coke ovens.

Coarse particles with a large particle diameter tend to crack at the contact interfaces due to the difference in shrinkage rate with adjacent coal during heating treatment, thus resulting in lower coke strength. On the other hand, fine particles with a small particle diameter tend to fly in the air when charged into the coke ovens, thus reducing bulk density. Grind conditions therefore must be adjusted so that the particle size after grinding of coal used for coke production is within a target particle size range so as to produce coke that has high strength and uniformity. Furthermore, while grind conditions can be changed based on coal properties, such as the particle size before grinding, or the particle size after grinding, it is important to measure the particle size of the coal before and after grinding.

In conventional coal particle size measurement, it has been proposed to calculate particle size distribution by identifying an image of coal in a two-dimensional image of the coal taken by a camera and calculating the particle diameter of each identified piece of coal (refer to Patent Literature [PTL] 1). Alternatively, a particle size measurement method has been proposed in which a laser beam is emitted onto coal and the particle diameter is calculated from an angle of the scattered laser beam (refer to Patent Literature [PTL] 2).

### CITATION LIST

### Patent Literatures

PTL 1: JP 2004-016983 A
PTL 2: JP 2012-173241 A

### SUMMARY

### (Technical Problem)

However, the particle size distribution calculation method proposed in PTL 1 does not include information in a depth direction, or in other words, in a height direction, of the image, and so when two or more particles are stacked, one particle in a lower layer cannot be identified. This makes it difficult to accurately measure particle size. In the particle size measurement method proposed in PTL 2, the particle size is calculated from the diffraction angle of the laser emitted onto the coal, so the larger the particle diameter, the larger the diffraction angle. This makes it difficult to measure particles with a large particle diameter, such as coal before grinding.

It would be helpful to provide a particle size estimation method and a particle size estimation device by which particle size is accurately estimated.

### (Solution to Problem)

A particle size estimation method according to the first aspect includes:
causing a plurality of particles to collide with predetermined equipment;
causing an acoustic sensor to collect sound of collision of the particles with the predetermined equipment; and
making an estimation regarding a particle size of the particles, based on the sound collected by the acoustic sensor.

A particle size estimation device according to the second aspect includes:
an acoustic sensor configured to be located in vicinity of predetermined equipment that handles a particle to be measured and configured to collect sound of collision when the particle collides with the predetermined equipment, wherein the particle includes a plurality of particles; and
an information processing unit configured to make an estimation regarding a particle size of the particles, based on the sound collected by the acoustic sensor.

### (Advantageous Effect)

According to the particle size estimation method and the particle size estimation device of the present disclosure configured as described above, an accurate estimation regarding the particle size of the particles can be made.

### BRIEF DESCRIPTION OF THE DRAWINGS

In the accompanying drawings:
FIG. 1 is a layout diagram illustrating a particle size estimation device with respect to predetermined equipment according to an embodiment;
FIG. 2 is a functional block diagram illustrating a schematic configuration of an information processing unit of FIG. 1;
FIG. 3 is a flowchart illustrating prior sampling processing executed by a control unit of FIG. 2;
FIG. 4 is a flowchart illustrating particle size estimation processing executed by the control unit of FIG. 2;
FIG. 5 includes the first graph, the second graph, and the third graph illustrating changes in sound pressure over time in a 0.5 kHz band of respective types of sound when coal with a particle diameter of 31.5 mm to 45 mm, that of 16 mm to 31.5 mm, and that of 16 mm to 45 mm collide with a hopper;
FIG. 6 includes the fourth graph and the fifth graph respectively illustrating changes in sound pressure over time in the 0.5 kHz band and a 14 kHz band of sound when coal with a particle diameter of 9 mm to 16 mm collides with the hopper; and
FIG. 7 includes the sixth graph and the seventh graph illustrating changes in sound pressure over time in the 14 kHz band of respective types of sound when coal with a particle diameter of 31.5 mm to 45 mm and that of 16 mm to 31.5 mm collide with the hopper.

### DETAILED DESCRIPTION

An embodiment of the present disclosure will be described below with reference to the drawings.

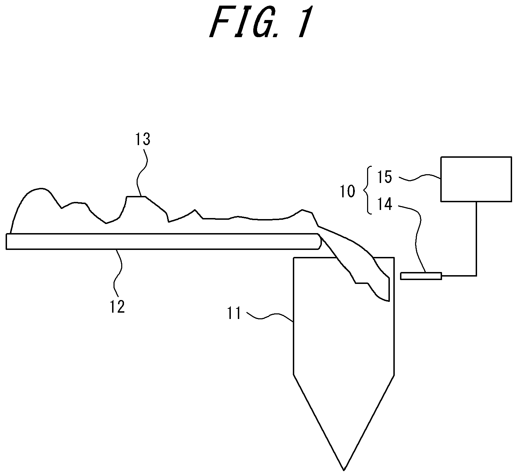

FIG. 1 is a schematic diagram illustrating a particle size estimation device 10 according to the embodiment of the present disclosure. The particle size estimation device 10 is provided in predetermined equipment 11 that handles a collection of a plurality of particles 13 to be measured. The plurality of particles includes, for example, coal that is greater than or equal to 5 mm. The predetermined equipment 11 is equipment with which the particles 13 are to collide. The predetermined equipment 11 is, for example, a hopper for storage. The plurality of particles 13 transported by a conveyor 12 is fed into the predetermined equipment 11 from vertically above and collides with a side surface of the predetermined equipment 11. The particle size estimation device 10 makes an estimation regarding the particle size of the plurality of particles 13, based on sound of collision between the predetermined equipment 11 and the plurality of particles 13.

The particle size estimation device 10 is configured to include an acoustic sensor 14 and an information processing unit 15.

The acoustic sensor 14 is located in the vicinity of the predetermined equipment. The acoustic sensor 14 is, for example, a microphone. The acoustic sensor 14 collects sound of collision when the plurality of particles 13 collides with the predetermined equipment 11. More specifically, the acoustic sensor 14 collects sound that includes sound of collision. It is assumed that the acoustic sensor 14 collects sound in a band in an audible range that is less than or equal to 20 kHz, and a sensor capable of detecting such sound may be selected.

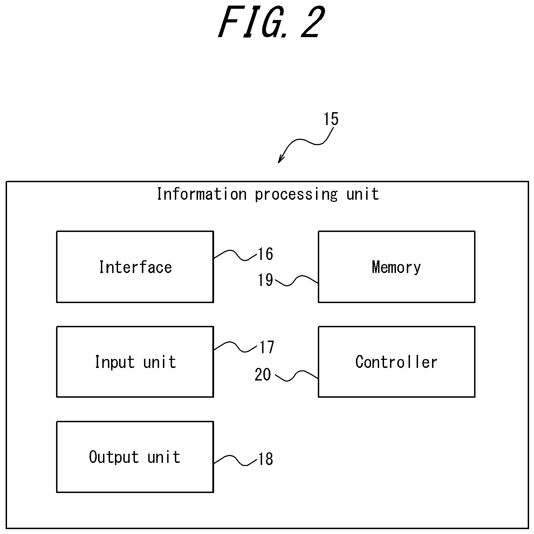

The information processing unit 15 may be a server unit or a personal computer. The information processing unit 15 makes an estimation regarding the particle size of the particles, based on the sound collected by the acoustic sensor 14. As illustrated in FIG. 2, the information processing unit 15 includes, for example, an interface 16, an input unit 17, an output unit 18, a memory 19, and a controller 20.

The interface 16 communicates information with external devices. The interface 16 acquires, for example, the sound collected by the acoustic sensor 14 as signals. The interface 16 may transmit and receive information to and from external devices other than the acoustic sensor 14.

The input unit 17 includes one or more interfaces that detect a user input. For example, the input unit 17 may include, but is not limited to, physical keys, capacitive keys, a touch screen integrally provided with a display of the output unit 18, a microphone that accepts audio input, and a camera that captures an image of a subject.

The output unit 18 includes one or more interfaces that output information and notify a user. For example, the output unit 18 may include, but is not limited to, a display that outputs video information and a speaker that outputs audio information.

The memory 19 may include, but is not limited to, semiconductor memory, magnetic memory, and optical memory. The memory 19 may function, for example, as main memory, auxiliary memory, or cache memory. The memory 19 stores any information used for operations of the information processing unit 15. For example, the memory 19 may store a system program, an application program, or the like. The memory 19 may store sound that corresponds to environmental sound of the predetermined equipment 11, as will be described later. The environmental sound of the predetermined equipment 11 is ambient sound, excluding the sound of collision when the plurality of particles 13 collides with the predetermined equipment 11, that is generated during the feeding of the plurality of particles 13 into the predetermined equipment 11.

The controller 20 includes one or more processors. Processors can be, but are not limited to, general-purpose processors or dedicated processors dedicated to specific processing. The dedicated processors may include application-specific integrated circuits (ASICs). The controller 20 may include a programmable logic device (PLD). The PLD may include a field-programmable gate array (FPGA). The controller 20 controls various parts of the information processing unit 15.

The controller 20 may include a prior sampling mode and a particle size estimation mode as operating modes. In the prior sampling mode, the controller 20 may sample environmental sound of the predetermined equipment 11. In the particle size estimation mode, the controller 20 may make an estimation regarding particle size. Switching between the prior sampling mode and the particle size estimation mode may be based on an operational input to the input unit 17.

In the prior sampling mode, a user of the particle size estimation device 10 is instructed to sample environmental sound of the predetermined equipment 11 in a state in which the particles 13 do not collide with it, before executing the estimation of the particle size. For example, the user is instructed to input, to the input unit 17, an operational input that causes the acoustic sensor 14 to collect sound in a state in which the conveyor 12 is in operation with nothing being placed on it.

In the prior sampling mode, the controller 20 may identify the sound collected by the acoustic sensor 14 as environmental sound and may store it in the memory 19. Furthermore, the controller 20 may separate the sound into a plurality of frequency bands by FFT analysis. The controller 20 may store a frequency band in the respective separated frequency bands in which the sound pressure is greater than or equal to a threshold in the memory 19 as a frequency band of the environmental sound.

In the particle size estimation mode, the user of the particle size estimation device 10 is instructed to cause the acoustic sensor 14 to collect sound while the particles 13 are colliding with the predetermined equipment 11. In the particle size estimation mode, the controller 20 makes an estimation regarding the particle size of the particles, based on the sound collected by the acoustic sensor 14, as will be described later.

For the estimation, the controller 20 may extract sound of collision of the particles from the sound collected by the acoustic sensor 14. The controller 20 may extract it, by cancelling the sound corresponding to the environmental sound of the predetermined equipment 11 stored in the memory 19 from the sound collected in the particle size estimation mode.

More specifically, in the particle size estimation mode, the controller 20 may separate the collected sound into a plurality of frequency bands, for example, by FFT analysis. The controller 20 may perform extraction, for example, by cancelling the environmental sound collected in the prior sampling mode by the sound separated into the plurality of frequency bands, or it may extract other frequency bands than the frequency band of the sound as the sound of collision of the particles.

The controller 20 may separate the collected sound into a plurality of frequency bands and make an estimation regarding the particle size of the particles, based on the sound of collision in the plurality of frequency bands. The controller 20 may make an estimation regarding the particle size of the particles, based on the extracted sound of collision of the particles, in other words, sound of collision in each of the plurality of frequency bands, excluding the environmental sound, as described above.

The controller 20 may, for example, determine whether the sound pressure in the first band in the plurality of separated frequency bands exceeds the first threshold. In a case in which the sound pressure in the first band exceeds the first threshold, the controller 20 may estimate that the plurality of particles contains particles 13 that are greater than or equal to the first particle diameter corresponding to the first band.

The first particle diameter may be freely determined. The first particle diameter may be, for example, the upper limit of a desired particle size range in the predetermined equipment 11. The first particle diameter is, for example, 31.5 mm. A frequency band in which changes in sound pressure are clearly demonstrated depending on whether the particles 13 to be measured exceed the determined first particle diameter may be determined as the first band. In a configuration in which the first particle diameter is 31.5 mm, the first band is the 0.5 kHz band. The first threshold is 0.05 m/s². The 0.5 kHz band is, for example, a band of a predetermined width centered at 0.5 kHz. The predetermined width may be zero. In other words, it may be 0.5 kHz itself or may have a width.

The controller 20 may also calculate, for example, the ratio of the maximum sound pressure value of sound of collision in the third band to the maximum sound pressure value of sound of collision in the second band in the plurality of separated frequency bands. The third band is a frequency range greater than the second band. The controller 20 may determine whether the ratio exceeds the second threshold. In a case in which the ratio exceeds the second threshold, the controller 20 may estimate that the plurality of particles contains only particles 13 that are less than or equal to the second particle diameter corresponding to the second band and the third band.

The second particle diameter may be freely determined. The second particle diameter may be, for example, the lower limit of the desired particle size range in the predetermined equipment 11. The second particle diameter is, for example, 16 mm. Frequency bands in which changes in the ratio of sound pressure are clearly demonstrated depending on whether the particles 13 to be measured exceed the determined second particle diameter may be determined as the second band and the third band. The second band may be the same as or different from the first band. In a configuration in which the second particle diameter is 16 mm, the second band and the third band are respectively the 0.5 kHz band and the 14 kHz band. The second threshold is 3. The 14 kHz band is, for example, a band of a predetermined width centered at 14 kHz. The predetermined width may be zero. In other words, it may be 0.5 kHz itself or may have a width.

After the estimation regarding the particle size of the particles, the controller 20 may cause the output unit 18 to output a result of the estimation. Alternatively, the controller 20 may supply the result of the estimation to an external device that performs predetermined processing on the particles 13 based on the estimated particle size.

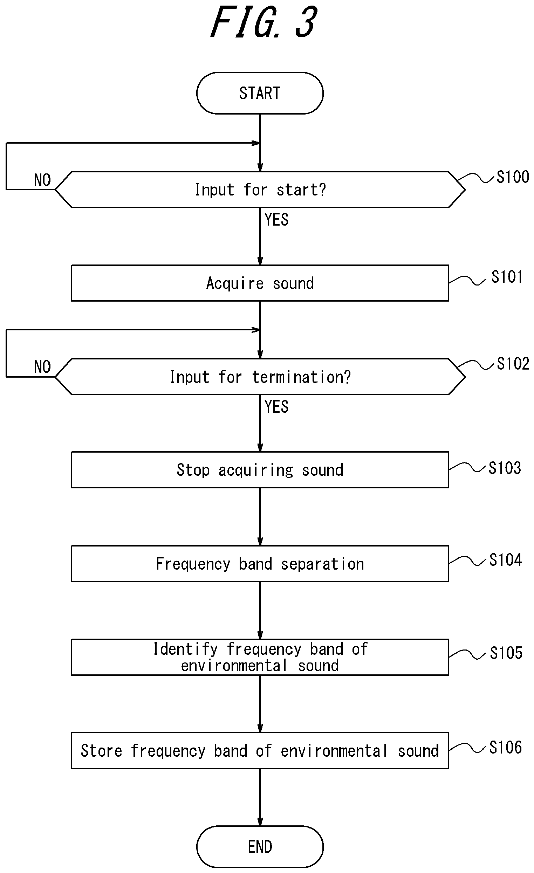

Next, prior sampling processing executed by the controller 20 in the present embodiment will be described with reference to the flowchart of FIG. 3. The prior sampling processing starts when the operating mode of the controller 20 is switched to the prior sampling mode.

In Step S100, the controller 20 determines whether the input unit 17 detects an operation input that causes the acoustic sensor 14 to start collecting sound. In a case in which it is not detected, the process returns to Step S100. In a case in which it is detected, the process proceeds to Step S101.

In Step S101, the controller 20 starts acquiring sound. The controller 20 starts storing the acquired sound, for example, in the memory 19. After the acquisition starts, the process proceeds to Step S102.

In Step S102, the controller 20 determines whether the input unit 17 detects an operation input that causes the acoustic sensor 14 to terminate the collection of sound. In a case in which it is not detected, the process repeats Step S102. In a case in which it is detected, the process proceeds to Step S103.

In Step S103, the controller 20 stops acquiring sound. After the stop, the process proceeds to Step S104.

In Step S104, the controller 20 separates the sound acquired from Step S101 to Step S103 into a plurality of frequency bands by FFT analysis. After the separation, the process proceeds to Step S105.

In Step S105, the controller 20 identifies a frequency band in the plurality of frequency bands separated in Step S104 in which the sound pressure as the amplitude is greater than or equal to a threshold, as a frequency band of environmental sound. After the identification, the process proceeds to Step S106.

In Step S106, the controller 20 stores the frequency band identified as the frequency band of the environmental sound in Step S105 in the memory 19. After the storing, the prior sampling process ends.

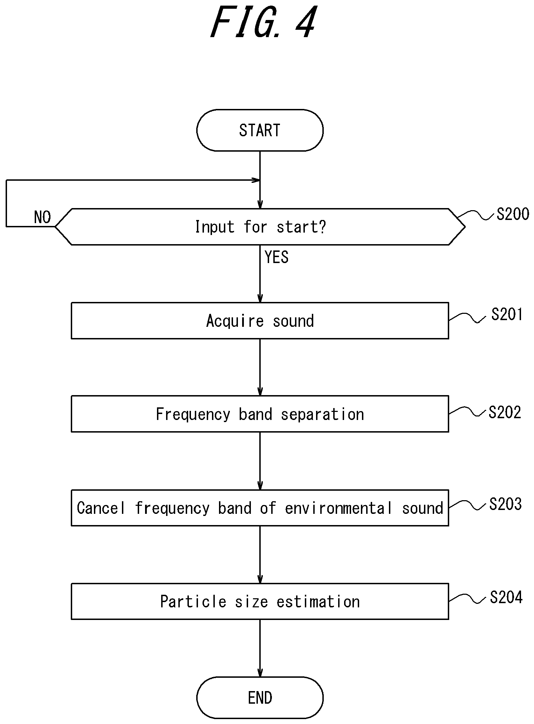

Next, particle size estimation processing executed by the controller 20 in the present embodiment will be described with reference to the flowchart of FIG. 4. The particle size estimation processing starts when the operating mode of the controller 20 is switched to the particle size estimation mode.

In Step S200, the controller 20 determines whether the input unit 17 detects an operation input that causes the acoustic sensor 14 to start collecting sound. In a case in which it is not detected, the process returns to Step S200. In a case in which it is detected, the process proceeds to Step S201.

In Step S201, the controller 20 starts acquiring sound. The controller 20 starts storing the acquired sound, for example, in the memory 19. After the acquisition starts, the process proceeds to Step S202.

In Step S202, the controller 20 separates the sound started to be acquired in Step S201 into a plurality of frequency bands by FFT analysis. After the separation, the process proceeds to Step S203.

In Step S203, the controller 20 cancels the frequency band of the environmental sound stored in the memory 19 from among the frequency bands separated in Step S202. After the cancellation, the process proceeds to Step S204.

In Step S204, the controller 20 makes an estimation regarding particle size of particles, based on sound in a plurality of frequency bands in which the environmental sound has been cancelled in Step S203. Furthermore, the controller 20 causes the output unit 18 to output a result of the estimation. Alternatively, the controller 20 supplies the result of the estimation to an external device. After the estimation regarding particle size, the particle size estimation processing ends.

The particle size estimation device 10 with the above configuration according to the present embodiment includes the acoustic sensor 14 that is located in the vicinity of the predetermined equipment 11 and that collects sound of collision when a plurality of particles 13 collides with the predetermined equipment 11, and it makes an estimation regarding the particle size of the particles, based on the sound collected by the acoustic sensor 14. Regarding sound of collision of particles, it is known that as the particle size of the particles decreases, the frequency band becomes higher and the sound pressure decreases. Hence, with the above configuration, the particle size estimation device 10 can accurately estimate the particle size of the particles.

Furthermore, the particle size estimation device 10 according to the present embodiment extracts sound of collision of the particles from the sound collected by the acoustic sensor 14. This configuration allows the particle size estimation device 10 to improve the accuracy of estimating the particle size because it extracts the sound of collision of the particles from the sound including sound that does not contribute to the estimation of the particle size.

Moreover, in the particle size estimation device 10 according to the present embodiment, the sound collected by the acoustic sensor 14 is separated into a plurality of frequency bands, and an estimation regarding the particle size of the particles is made based on respective types of sound of collision in the plurality of separated frequency bands. Because there are differences in sound pressure between different frequency bands depending on the particle size of the particles, the above configuration allows the particle size estimation device 10 to make estimations in accordance with a variety of particle sizes.

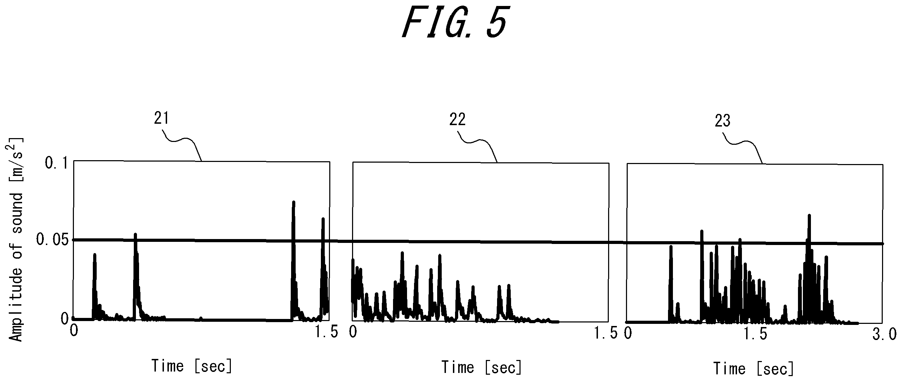

Moreover, in a case in which the sound pressure in the first band in the plurality of separated frequency bands exceeds the first threshold, the particle size estimation device 10 estimates that the plurality of particles contains particles 13 that are greater than or equal to the first particle diameter corresponding to the first band. Specifically, the effect of the above configuration will be illustrated using actual measurements of sound pressure in the 0.5 kHz band for a particle diameter of around 31.5 mm. As illustrated in FIG. 5, in the first graph 21 representing changes in sound pressure over time in the 0.5 kHz band of sound when coal with a particle diameter of 31.5 mm to 45 mm collides with the hopper, the peak value of the sound pressure exceeds 0.05. In the second graph 22 representing changes in sound pressure over time in the 0.5 kHz band of sound when coal with a particle diameter of 16 mm to 31.5 mm collides with the hopper, the peak value of the sound pressure does not exceed 0.05. In the third graph 23 representing changes in sound pressure over time in the 0.5 kHz band of sound when coal with a particle diameter of 16 mm to 45 mm collides with the hopper, the peak value of the sound pressure exceeds 0.05. Based on these actual measurements, it is possible to estimate that the particle diameter of the coal to be measured exceeds 31.5 mm in a case in which the sound pressure in the 0.5 kHz band exceeds 0.05. Thus, the particle size estimation device 10 with the above configuration can estimate with high accuracy that the plurality of particles contains particles 13 that are greater than or equal to the first particle diameter, by setting the first band and the first threshold in accordance with the actual measurements for the predetermined equipment 11.

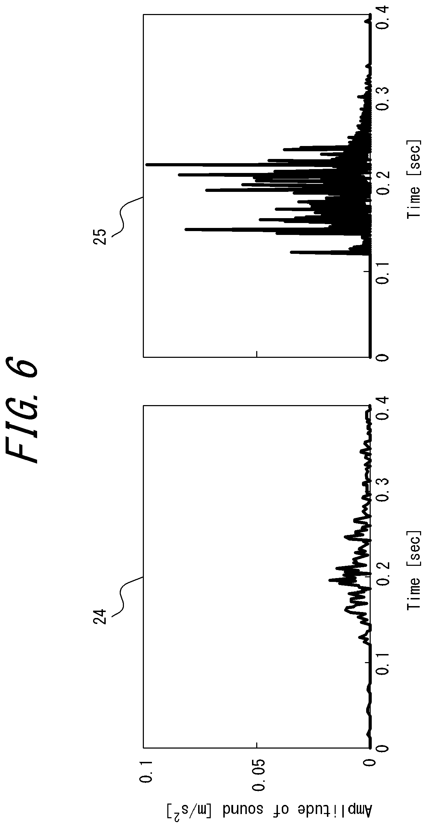

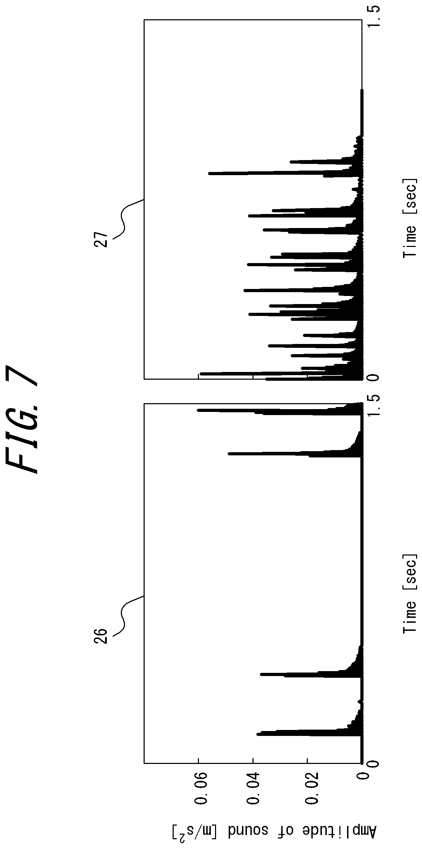

Moreover, in a case in which the ratio of the maximum sound pressure value of sound of collision in the third band that is greater than the second band to the maximum sound pressure value of sound of collision in the second band in the plurality of separated frequency bands exceeds the second threshold, the particle size estimation device 10 according to the present embodiment estimates that the plurality of particles contains only particles 13 that are less than or equal to the second particle diameter corresponding to the second band and the third band. Specifically, the effect of the above configuration will be illustrated using actual measurements of sound pressure in the 0.5 kHz band and the 14 kHz band for a particle diameter of around 16 mm. As illustrated in FIG. 6, in the fourth graph 24 representing changes in sound pressure over time in the 0.5 kHz band of sound when coal with a particle diameter of 9 mm to 16 mm collides with the hopper, the peak value of the sound pressure is approximately 0.02. In the fifth graph 25 representing changes in sound pressure over time in the 14 kHz band of sound when coal with a particle diameter of 9 mm to 16 mm collides with the hopper, the peak value of the sound pressure is approximately 0.1. As illustrated in FIG. 7, in the sixth graph 26 representing changes in sound pressure over time in the 14 kHz band of sound when coal with a particle diameter of 31.5 mm to 45 mm collides with the hopper, the peak value of the sound pressure is approximately 0.05. In the seventh graph 27 representing changes in sound pressure over time in the 14 kHz band of sound when coal with a particle diameter of 16 mm to 31.5 mm collides with the hopper, the peak value of the sound pressure is approximately 0.05. As can be seen from the first graph 21 to the seventh graph 27, the ratio of the peak of sound pressure in a high frequency band to a low frequency band increases as the particle size decreases. Thus, the particle size estimation device 10 with the above configuration can estimate with high accuracy that the plurality of particles contains only particles 13 that are less than or equal to the second particle diameter, by setting the second band, the third band, and the second threshold in accordance with the actual measurements for the predetermined equipment 11.

An embodiment of the particle size estimation device 10 has been described. The embodiment of the present disclosure can, however, also be embodied as a method or a program for implementing the device, or a storage medium in which the program is recorded (examples may include an optical disc, an optical-magnetic disk, a CD-ROM, a CD-R, a CD-RW, a magnetic tape, a hard disk, and a memory card).

The implementation form of the program is not limited to an application program, such as object code compiled by a compiler or a program code executed by an interpreter, but can also take the form of a program module or the like that is embedded in an operating system. Furthermore, the program may or may not be configured so that all processing is implemented only in a CPU on a control board. The program may be configured to be implemented in whole or in part by another processing unit mounted on an expansion board or an expansion unit added to the board as needed.

The figures illustrating the embodiment of the present disclosure are schematic. Dimensional ratios or the like on the drawings do not necessarily match actual ones.

While the embodiment of the present disclosure has been described based on the drawings and examples, it is to be noted that various changes and alterations may be made by those skilled in the art based on the present disclosure. Accordingly, such changes and alterations are included within the scope of the present disclosure. For example, functions or the like included in each component or the like can be rearranged in a logically consistent manner, and a plurality of components or the like can be combined into one or divided.

All the constituent features described in the present disclosure and/or all the methods or all the steps of processing disclosed can be combined in any combination, unless these features included in the combination are mutually exclusive. Each of the features described in the present disclosure may also be replaced by alternative features that serve for the same purposes, equivalent purposes, or similar purposes, unless explicitly denied. Each of the disclosed features, therefore, is just an example of a comprehensive set of the same or equivalent features, unless explicitly denied.

Furthermore, the embodiment according to the present disclosure is not limited to any of the specific configurations in the embodiment described above. The embodiment according to the present disclosure may be extended to all the novel features described in the present disclosure or any combination thereof, or all the novel methods or steps of processing described in the present disclosure or any combination thereof.

In the present disclosure, the terms, such as the "first" or the "second," are identifiers for distinguishing the constitutions. The constitutions distinguished by the terms, such as the "first" or the "second," in the present disclosure may exchange the numbers thereof. For example, the "first" as an identifier of the first band and the "second" as an identifier of the second band may be exchanged. The identifiers may simultaneously be exchanged. Even after the identifiers are exchanged, the constitutions are still distinguished. The identifiers may be deleted. Constitutions from which the identifiers have been removed are distinguished by reference numerals. The identifier terms, such as the "first" or the "second," in the present disclosure are not intended to be used as a basis for interpretation of order of the constitutions or the presence of an identifier with a smaller number.

### REFERENCE SIGNS LIST

- 10: Particle size estimation device
- 11: Hopper
- 12: Conveyor
- 13: Plurality of particles
- 14: Acoustic sensor
- 15: Information processing unit
- 16: Interface
- 17: Input unit
- 18: Output unit
- 19: Memory
- 20: Controller
- 21: First graph
- 22: Second graph
- 23: Third graph
- 24: Fourth graph
- 25: Fifth graph
- 26: Sixth graph
- 27: Seventh graph

## Claims

1. A particle size estimation method, comprising:
causing a plurality of particles to collide with predetermined equipment;
causing an acoustic sensor to collect sound of collision of the particles with the predetermined equipment; and
making an estimation regarding a particle size of the particles, based on the sound collected by the acoustic sensor.

2. The particle size estimation method according to claim 1, wherein the estimation regarding the particle size of the particles includes
extracting sound of collision of the particles from the sound collected by the acoustic sensor.

3. The particle size estimation method according to claim 2, wherein the sound of collision of the particles is extracted, by separating sound corresponding to environmental sound into a plurality of frequency bands and cancelling the sound corresponding to the environmental sound from the sound collected by the acoustic sensor.

4. The particle size estimation method according to any one of claims 1 to 3, wherein the estimation regarding the particle size of the particles includes:
separating the sound collected by the acoustic sensor into a plurality of frequency bands; and
making an estimation regarding the particle size of the particles, based on sound of collision in which environmental sound separated into the plurality of frequency bands is cancelled, in each of the plurality of separated frequency bands.

5. The particle size estimation method according to claim 4, wherein the estimation regarding the particle size of the particles includes
in a case in which sound pressure in a first band in the plurality of separated frequency bands exceeds a first threshold, estimating that the plurality of particles contains particles that are greater than or equal to a first particle diameter corresponding to the first band.

6. The particle size estimation method according to any one of claims 2 to 4, wherein the estimation regarding the particle size of the particles includes
in a case in which a ratio of a maximum sound pressure value of sound of collision in a third band that is greater than a second band to a maximum sound pressure value of sound of collision in the second band in a plurality of separated frequency bands exceeds a second threshold, estimating that the plurality of particles contains only particles that are less than or equal to a second particle diameter corresponding to the second band and the third band.

7. The particle size estimation method according to any one of claims 1 to 6, wherein the sound collected by the acoustic sensor is in a band in an audible rage that is less than or equal to 20 kHz.

8. The particle size estimation method according to any one of claims 1 to 7, wherein the plurality of particles contains coal that is greater than or equal to 5 mm.

9. A particle size estimation device comprising:
an acoustic sensor configured to be located in vicinity of predetermined equipment that handles a particle to be measured and configured to collect sound of collision when a plurality of the particles collides with the predetermined equipment; and
an information processing unit configured to make an estimation regarding a particle size of the particles, based on the sound collected by the acoustic sensor.
